# EUROPÄISCHE PATENTSCHRIFT

(11) **EP 4 162 903 B1**
(45) Veröffentlichungstag und Bekanntmachung des Hinweises auf die Patenterteilung: **14.02.2024**
(21) Anmeldenummer: 22199154.0
(22) Anmeldetag: 30.09.2022
(51) Int. Cl.: A61F 2/30, A61F 2/44, A61F 2/46

(54) **AUFSPREIZBARES ZWISCHENWIRBELIMPLANTAT**
EXPANDABLE INTERVERTEBRAL IMPLANT
IMPLANT INTERVERTÉBRAL EXPANSIBLE

(30) Priorität: 05.10.2021 DE 202021105393 U
(43) Veröffentlichungstag der Anmeldung: 12.04.2023
(73) Patentinhaber: Richard Wolf GmbH, 75438 Knittlingen (DE)
(72) Erfinder: Pothof, Frederick, 76646 Bruchsal (DE); Göthel, Dirk, 75057 Kürnbach (DE)
(74) Vertreter: Patentanwälte Hemmer Lindfeld Frese Partnerschaft mbB

(56) Entgegenhaltungen:
- US-A1- 2013 006 361
- US-A1- 2015 012 097
- US-A1- 2015 342 748

## Beschreibung

Die vorliegende Offenbarung betrifft ein aufspreizbares Zwischenwirbelimplantat insbesondere zum Einsatz in der Humanmedizin. Ein solches Zwischenwirbelimplantat ist zum Beispiel im Patentdokument US2015/0342748 offenbart.

Solche aufspreizbaren Zwischenwirbelimplantate werden in der Humanmedizin zur intrakorporalen Fusion (Versteifung der Wirbelkörper) bei Wirbelsäulenerkrankungen eingesetzt. Das Zwischenwirbelimplantat dient also der Fusion zweier Wirbelkörper, insbesondere im lumbalen Bereich der Wirbelsäule. Dabei wird zunächst der Zwischenwirbelraum von dorsal ausgeräumt und das Zwischenwirbelimplantat eingeführt, um die Wirbelkörper auf Abstand und ggf. in einer bestimmten Winkelposition zueinander (Lordose) zu halten.

Einerseits muss das Zwischenwirbelimplantat möglichst klein sein, damit es mittels eines endoskopischen Instruments möglichst minimalinvasiv in den Zwischenwirbelraum eingebracht werden kann. Andererseits muss die Aufspreizkraft möglichst flächig verteilt werden, um das Risiko eines Einbrechens des Implantats in den Wirbelkörper zu verringern.

Aus dem Stand der Technik bekannte Zwischenwirbelimplantate weisen relativ komplizierte Spreizmechanismen mit Hebel, Verschwenkungen und relativ vielen Einzelteilen auf.

Aufgabe der vorliegenden Offenbarung ist es, ein minimalinvasiv endoskopisch in den Zwischenwirbelraum einbringbares Aufspreizbares Zwischenwirbelimplantat bereitzustellen, welches es dem Wirbelknochen erleichtert, in das Implantat hineinzuwachsen und/oder dieses zu umkapseln, wobei das Risiko des Einbrechens des Implantats in den Wirbelkörper reduziert ist. Zudem soll das Zwischenwirbelimplantat möglichst wenig Einzelteile und eine möglichst unkomplizierte Spreizmechanik aufweisen. Die Erfindung ist im unabhängigen Anspruch definiert. Besondere Ausführungsarten sind in den abhängigen Ansprüchen enthalten.

Zur Lösung des oben genannten Problems wird gemäß der vorliegenden Offenbarung ein aufspreizbares Zwischenwirbelimplantat bereitgestellt mit zwei relativ zueinander in einer Spreizrichtung beweglichen Knochenanlageelementen, einem zwischen den Knochenanlagenelementen angeordneten Zwischenrahmen, einem innerhalb des Zwischenrahmens in einer quer zur Spreizrichtung verlaufenden Klemmrichtung beweglichen Aufspreizschlitten, und einem in Klemmrichtung durchgehend durch den Aufspreizschlitten verlaufenden Aktuatorschaft. Der Aufspreizschlitten weist dabei erste Gleitflächen auf, welche schräg zur Klemmrichtung verlaufen. Jedes der Knochenanlageelemente weist mindestens eine zu einer der ersten Gleitflächen korrespondierende zweite Gleitfläche auf, welche parallel zu der korrespondierenden ersten Gleitfläche verläuft und bei Klemmrichtungsbewegung des Aufspreizschlittens auf dieser gleitet. Der Aktuatorschaft ist an einer Frontseite des Zwischenrahmens und an einer Rückseite des Zwischenrahmens um die Klemmrichtung drehbar gelagert und mit dem Aufspreizschlitten derart formschlüssig gekoppelt, dass sich der Aufspreizschlitten bei Drehung des Aktuatorschafts zwischen der Frontseite des Zwischenrahmens und der Rückseite des Zwischenrahmens in Klemmrichtung bewegt.

Es ist daher möglich, das aufspreizbare Zwischenwirbelimplantat aus fünf oder sechs Einzelteilen zu fertigen, ohne dass eine komplizierte Spreizmechanik benötigt wird. Das aufspreizbare Zwischenwirbelimplantat kann daher besonders kompakt für das minimal-invasive Einbringen mittels eines endoskopischen Instruments ausgestaltet werden. Zugleich wird durch die parallel zueinander verlaufenden korrespondierenden Gleitflächen eine Rotation der Knochenanlageelemente verhindert, und dadurch punktuelle Belastungen des Wirbelkörperknochens vermieden und somit das Risiko des Einbrechens des Implantats in den Wirbelkörper verringert wird. Die parallelen korrespondierenden Gleitflächen führen also zu einer möglichst rotationsfreien Parallelverschiebung der Knochenanlageelemente und somit zu einer möglichst homogenen Kraftverteilung über einen möglichst großen Bereich der Knochenanlageelemente.

Optional können die ersten Gleitflächen in einem Winkel α von 10° bis 45° schräg zur Klemmrichtung verlaufen. Je flacher der Winkel gewählt wird, um so kleiner ist das Übersetzungsverhältnis von der Bewegung des Aufspreizschlittens in Klemmrichtung zur Bewegung der Knochenanlageelemente in Spreizrichtung. Je größer der Winkel gewählt wird, umso weiter lässt sich das Wirbelsäulenimplantat mit einer relativ kurzen Bewegung des Aufspreizschlittens in Klemmrichtung aufspreizen. Je nach Anwendungserfordernis können verschiedene Modelle des Zwischenwirbelimplantats bereitgestellt werden mit entsprechend steilem oder flachem Winkel der Gleitflächen.

Optional kann der Aktuatorschaft mit einem selbsthemmenden Gewinde form- und kraftschlüssig mit dem Aufspreizschlitten gekoppelt sein. Durch ein solches selbsthemmendes Gewinde kann eine Drehbewegung des Aktuatorschafts, beispielsweise mittels eines Schraubendrehers, in eine lineare Bewegung des Aufspreizschlittens in Klemmrichtung umgesetzt werden. Der Bewegungsspielraum des Aufspreizschlittens innerhalb des Zwischenrahmens verläuft zwischen einer Innenseite der Frontseite des Zwischenrahmens und einer Innenseite der Rückseite des Zwischenrahmens.

Optional können die Knochenanlageelemente ausschließlich linear und rotationslos in der Spreizrichtung beweglich sein. Es gibt also außer dem Aktuatorschaft keine drehenden Elemente, sodass die Knochenanlageelemente eine reine Parallelverschiebung erfahren, was das Risiko von punktuellen Belastungen des Wirbelkörpers reduziert.

Optional können die Knochenanlageelemente separate Bauteile sein, welche vom Aufspreizschlitten in einem durch eine Drehstellung des Aktuatorschafts definierten Abstand in Spreizrichtung zueinander gehalten werden. Es ist also keine Verbiegung oder Verformung von Bauteilen notwendig, um das Zwischenwirbelimplantat aufspreizen zu können. Dies eröffnet größere Freiheiten bei der Materialwahl und den Herstellungsverfahren, insbesondere für die Knochenanlageelemente.

Optional kann der Aufspreizschlitten von den ersten Gleitflächen weg gerichtete dritte Gleitflächen aufweisen, wobei jedes der Knochenanlageelemente mindestens eine zu einer der dritten Gleitflächen korrespondierende vierte Gleitfläche aufweist, welche parallel zu der korrespondierenden dritten Gleitfläche verläuft und bei Bewegung des Aufspreizschlittens entgegen der Klemmrichtungsbewegung auf dieser gleitet. Das Zwischenwirbelimplantat kann dadurch je nach Drehrichtung des Aktuatorschafts nicht nur aufgespreizt werden, sondern auch wieder zusammengezogen werden. Die miteinander korrespondierenden dritten und vierten Gleitflächen ziehen also die Knochenanlageelemente in Spreizrichtung wieder zueinander, wenn der Aktuatorschaft in die entsprechende Drehrichtung gedreht wird. Dies erlaubt Korrekturen beim Einbringen des Zwischenimplantats.

Optional können die Knochenanlageelemente jeweils im Zwischenrahmen in Spreizrichtung geführt gelagert sein. Vorzugsweise umschließt der Zwischenrahmen in einer zur Spreizrichtung orthogonalen Ebene den Aufspreizschlitten vollständig umlaufend und weist Innenflächen auf, an welchen die Knochenanlageelemente geführt in Spreizrichtung gleiten können.

Optional kann die Frontseite des Zwischenrahmens eine erste Aktuatorschaftaufnahmeöffnung aufweisen und die Rückseite des Zwischenrahmens entsprechend eine zweite Aktuatorschaftaufnahmeöffnung. Der Aktuatorschaft kann sich dadurch der Länge nach in Klemmrichtung durch den Zwischenrahmen erstrecken und den Aufspreizschlitten im Zwischenrahmen sichern.

Optional kann der Aktuatorschaft von der Frontseite des Zwischenrahmens aus in den Zwischenrahmen einsteckbar sein und ein durch die Rückseite des Zwischenrahmens ragendes Ende des Aktuatorschafts kann mit einem Sicherungsmittel, beispielsweise einer Mutter, einem Sicherungsring oder einem Sicherungsstift, an der Rückseite des Zwischenrahmens gesichert sein. Der Aktuatorschaft kann bolzenförmig ausgestaltet und mit einem Bolzenkopf an der Frontseite des Zwischenrahmens gesichert sein.

Optional kann die Rückseite des Zwischenrahmens beidseitig lateral und/oder zu den Knochenanlageelementen hin schräg angefast sein. Dies ist sinnvoll, da das Zwischenwirbelimplantat vorzugsweise mit der Rückseite des Zwischenrahmens voraus in den Zwischenwirbelraum eingeführt wird und sich möglichst nicht verhaken oder verkanten soll.

Optional kann der Zwischenrahmen eine erste laterale Seite aufweisen, die von der Frontseite zur Rückseite verläuft, sowie eine der ersten lateralen Seite gegenüberliegende zweite laterale Seite, die ebenfalls von der Frontseite zur Rückseite verläuft, wobei die erste laterale Seite in Klemmrichtung kürzer ist als die zweite laterale Seite. Das Zwischenwirbelimplantat ist also vorzugsweise bezüglich einer von der Spreizrichtung und der Klemmrichtung aufgespannten Ebene nicht spiegelsymmetrisch ausgestaltet, sondern auf einer lateralen Seite kürzer als auf der anderen lateralen Seite. Dies erlaubt es, das Zwischenwirbelimplantat über einen medio-lateralen Zugang, also schräg von der Seite in den Zwischenwirbelraum zu platzieren und den Kontaktbereich der Knochenanlageelemente mit dem Wirbelkörper zu maximieren.

Optional können sich die Knochenanlageelemente jeweils zur Rückseite des Zwischenrahmens hin keilförmig verdicken. Das Zwischenwirbelimplantat kann somit an eine bestimmte zu erzielende Lordose, insbesondere im lumbalen Wirbelsäulenbereich, angepasst sein. Es können verschiedene Modelle des Zwischenwirbelimplantats bereitgestellt werden, die für eine jeweils zu erzielende Lordose angepasst sind.

Optional können zumindest die Knochenanlageelemente, vorzugsweise sämtliche Elemente des Zwischenwirbelimplantats, jeweils durch ein generatives Schichtaufbauverfahren, vorzugsweise selektives Lasersintern, SLS, aus Titan oder einem titanhaltigen Material hergestellt sein. Dies hat den Vorteil, dass eine raue Oberfläche bereitgestellt werden kann, welche es dem Knochen erleichtert, in das Implantat hineinzuwachsen und/oder dieses zu umkapseln. Außerdem können sehr stabile feine Strukturen, Profile und/oder Durchbrechungen in den Knochenanlageelementen durch 3D-Druck erzielt werden, in welche der Wirbelkörperknochen besonders gut einwachsen kann. Ein generatives Schichtaufbauverfahren hat zudem den Vorteil, dass Zwischenwirbelimplantate patientenspezifisch als Unikat hergestellt werden können, um patientenspezifischen Besonderheiten zu begegnen.

Optional können die Knochenanlageelemente jeweils eine Knochenanlageseite aufweisen, wobei die Knochenanlageseite ein Profil und/oder Löcher aufweist. Die Knochenanlageseiten der Knochenanlageelemente weisen vorzugsweise voneinander weg und erstrecken sich über einen möglichst großen Bereich der Knochenanlageelemente. Die Knochenanlageelemente sind vorzugsweise plattenförmig ausgestaltet. Das Profil und/oder die Löcher in der jeweiligen Knochenanalageseite führen zu einem Formschluss mit dem Wirbelkörperknochen, sobald dieser in das Zwischenwirbelimplantat eingewachsen ist, bzw. dieses umwachsen hat. Der Zwischenrahmen ragt vorzugsweise lateral nicht über die Knochenanlageseiten der Knochenanlageelemente hinaus.

Optional können die ersten Gleitflächen spiegelsymmetrisch zu einer quer zur Spreizrichtung verlaufenden Ebene angeordnet sein. Dies ist vorteilhaft, um beide Knochenanlageelemente relativ zum Zwischenrahmen durch Drehung des Aktuatorschafts gleich schnell in Spreizrichtung zu bewegen.

Optional kann eine Hälfte der ersten Gleitflächen an einer Oberseite des Aufspreizschlittens angeordnet sein und eine andere Hälfte der ersten Gleitflächen an einer Unterseite des Aufspreizschlittens. Dies erlaubt eine besonders einfache Ausgestaltung des Aufspreizschlittens.

Optional kann eine Hälfte der ersten Gleitflächen an einer ersten lateralen Seite des Aufspreizschlittens angeordnet sein und eine andere Hälfte der ersten Gleitflächen an einer der ersten lateralen Seite gegenüberliegenden zweiten lateralen Seite des Aufspreizschlittens angeordnet sein. Dies hat den Vorteil, dass sich die Knochenanlageelemente nicht im Zwischenrahmen verkanten und der Aufspreizschlitten die beiden Knochenanlageelemente gleichzeitig mittels mehrerer erster Gleitflächen bewegt. Vorzugsweise weist der Aufspreizschlitten insgesamt acht oder zwölf erste Gleitflächen auf, von denen die Hälfte parallel zueinander angeordnete erste Gleitflächen mit entsprechenden zweiten Gleitflächen des ersten Knochenanlageelements korrespondieren und die andere Hälfte der jeweils zueinander parallel verlaufenden ersten Gleitflächen mit entsprechenden zweiten Gleitflächen des zweiten Knochenanlageelements korrespondieren. Der Aufspreizschlitten ist vorzugsweise im Wesentlichen sowohl spiegelsymmetrisch bezüglich einer von der Spreizrichtung und der Klemmrichtung aufgespannten Ebene als auch bezüglich einer senkrecht zur Spreizrichtung verlaufenden Ebene.

Optional können die zweiten Gleitflächen durch innenseitig schräg in lateralen Randleisten der Knochenanlageelemente verlaufenden Nuten ausgebildet sein. Diese Nuten können vorzugsweise nicht nur die zweiten Gleitflächen bilden, sondern auch die vierten Gleitflächen, sodass durch die Nuten die Knochenanlageelemente nicht nur auseinander bewegt werden können, sondern auch durch entsprechende Drehrichtung des Aktuatorschaft wieder zueinander werden können. Der Aufspreizschlitten kann entsprechende Stege aufweisen, welche in die Nuten der Knochenanlageelemente eingreifen und die ersten und vorzugsweise auch die dritten Gleitflächen bilden.

Optional können die lateralen Randleisten der Knochenanlageelemente als Führungselement außenseitig passgenau in den Zwischenrahmen eingreifen. Dadurch sind die Knochenanlageelemente in besonders einfacher Weise im Zwischenrahmen derart geführt, dass sie sich nur linear in Spreizrichtung bewegen können.

Das hierin offenbarte aufspreizbare Zwischenwirbelimplantat wird nun anhand der beiliegenden Figuren näher erläutert. Es zeigen:
- Fig. 1: eine perspektivische Ansicht eines Ausführungsbeispiels eines hierin offenbarten Zwischenwirbelimplantats bei eingefahrenen Knochenanlageelementen;
- Fig. 2 bis 5: verschiedene Explosionsansichten des in Fig. 1 gezeigten Ausführungsbeispiels; und
- Fig. 6: eine orthografische Explosionsansicht eines weiteren Ausführungsbeispiels eines hierin offenbarten Zwischenwirbelimpla ntats.

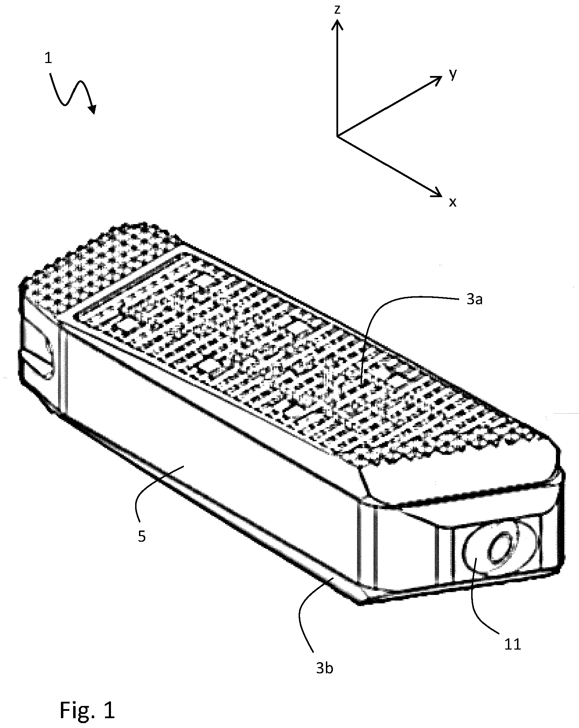

In Fig. 1 ist ein Ausführungsbeispiel des hierin offenbaren aufspreizbaren Zwischenwirbelimplantats 1 in eingefahrenem Zustand gezeigt. In diesem eingefahrenen Zustand ist das Zwischenwirbelimplantat 1 besonders kompakt und kann über einen Arbeitskanal eines endoskopischen Instruments (nicht gezeigt), vorzugsweise unter endoskopischer Sicht, in einen zuvor ausgeräumten Zwischenwirbelraum eingebracht werden. Zur besseren Orientierung zeigt Fig. 1 ein rechtshändiges kartesisches Koordinatensystem, wobei die x-Richtung in einer Längsachse des Zwischenwirbelimplantats 1 verläuft, die y-Richtung in einer Querachse des Zwischenimplantats 1, und die z-Achse in einer Vertikalrichtung des Zwischenimplantats 1. Das Zwischenwirbelimplantat 1 wird typischerweise in positive x-Richtung in einen Zwischenwirbelraum rückwärts eingeführt. Die in Fig. 1 gewählte Ansicht auf das Zwischenwirbelimplantat 1 ist also schräg von hinten. Das Zwischenwirbelimplantat 1 ist in z-Richtung aufspreizbar, d.h. eine Spreizrichtung verläuft in z-Richtung.

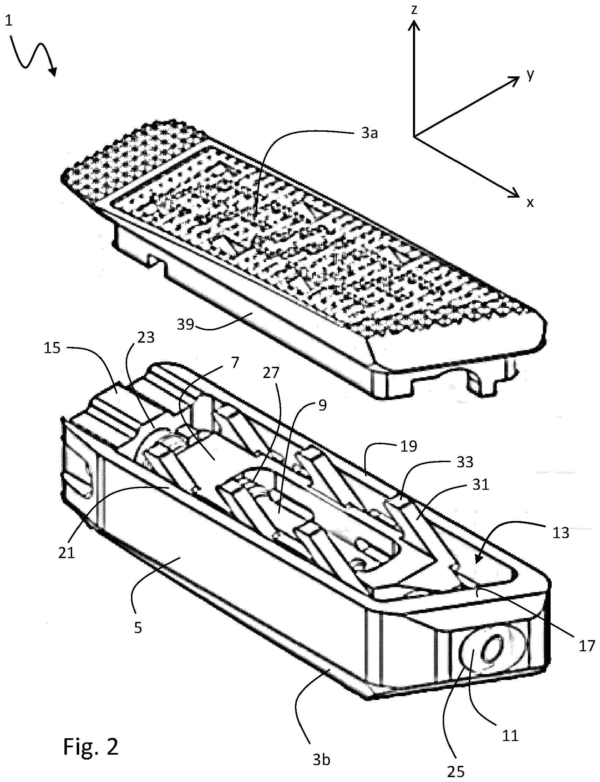

Das Zwischenwirbelimplantat 1 besteht in dem gezeigten Ausführungsbeispiel aus sechs Einzelteilen. Dies sind zwei relativ zueinander in der Spreizrichtung z bewegliche Knochenanlageelemente 3a,b, ein zwischen den Knochenanlageelementen 3a,b angeordneter Zwischenrahmen 5, ein innerhalb des Zwischenrahmens 5 in einer quer zur Spreizrichtung z verlaufenden Klemmrichtung x beweglichen Aufspreizschlitten 7 (nur in Fig. 2 bis 6 sichtbar), ein in Klemmrichtung x durchgehend durch den Aufspreizschlitten 7 verlaufender Aktuatorschaft 9 (ebenfalls nur in Fig. 2 bis 6 sichtbar) und ein Sicherungsmittel 11 in Form einer Mutter.

Fig. 2 zeigt eine Explosionsansicht des Zwischenwirbelimplantats 1, beim dem das obere Knochenanlageelement 3a abgehoben dargestellt ist. Dadurch wird die Sicht auf den sich im Zwischenrahmen 5 befindlichen Aufspreizschlitten 7 sowie auf den Aktuatorschaft 9 ermöglicht. Der Zwischenrahmen 5 ist ein in der xy-Ebene vollständig geschlossen umlaufender Rahmen, der den Aufspreizschlitten 7 vollständig einrahmt. Dadurch definiert der Zwischenrahmen 5 einen nach oben und unten offenen Innenraum 13. Nach vorne hin, also in negative x-Richtung, wird der Innenraum 13 durch eine Frontseite 15 des Zwischenrahmens 5 begrenzt. Nach hinten hin, also in positive x-Richtung, wird der Innenraum 13 durch eine Rückseite 17 des Zwischenrahmens 5 begrenzt. Laterale Seiten 19, 21 des Zwischenrahmens 5 begrenzen den Innenraum 13 in laterale Richtung, d.h. in y-Richtung. Der Aufspreizschlitten 7 passt mit seiner Breite in y-Richtung passgenau in den Innenraum 13, sodass die lateralen Seiten 19, 21 eine Drehung des Aufspreizschlittens 7 um die x-Achse verhindern. Die Länge des Aufspreizschlittens 7 in x-Richtung ist allerdings kürzer als die Länge des Innenraums 13 in x-Richtung, damit sich der Aufspreizschlitten 7 innerhalb des Zwischenrahmens 5 in die Klemmrichtung x in Grenzen bewegen kann.

Der Aktuatorschaft 9 erstreckt sich der Länge nach in Klemmrichtung x durch die Frontseite 15 des Zwischenrahmens 5, vollständig der Länge nach durch den Aufspreizschlitten 7, und durch die Rückseite 17 des Zwischenrahmens 5. Dazu weist die Frontseite 15 des Zwischenrahmens 5 eine erste Aktuatorschaftaufnahmeöffnung 23 auf und die Rückseite 17 des Zwischenrahmens 5 eine zweite Aktuatorschaftaufnahmeöffnung 25. Der Aktuatorschaft 9 ist drehbar in der ersten und zweiten Aktuatorschaftaufnahmeöffnung 23, 25 gelagert und kann von der Frontseite 15 des Zwischenrahmens 5 aus beispielsweise mit einem Schraubendreher um die Klemmrichtung x gedreht werden.

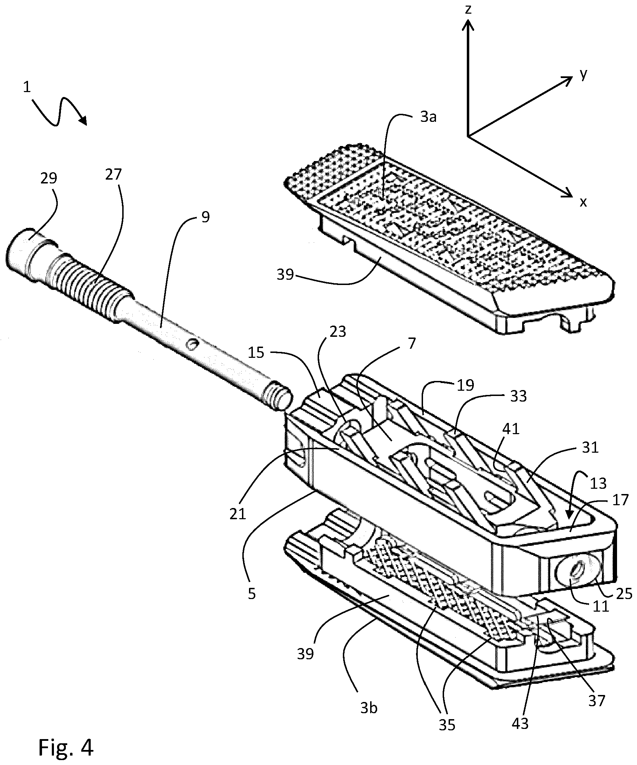

Der Aktuatorschaft 9 ist mit einem selbsthemmenden Gewinde 27 form- und kraftschlüssig mit dem Aufspreizschlitten 7 gekoppelt. Dazu weist der Aufspreizschlitten 7 ein korrespondierendes Innengewinde auf, in welches der Aktuatorschaft 19 eingeschraubt ist. Der Aktuatorschaft 9 ist im Gegensatz zum Aufspreizschlitten 7 allerdings in Klemmrichtung x relativ zum Zwischenrahmen 5 fixiert. In positive x-Richtung weist der Aktuatorschaft 9 dazu an der Frontseite 15 des Zwischenrahmens 5 einen Bolzenkopf 29 (nur in Fig. 4 bis 6 sichtbar) auf. Gegen eine Bewegung des Aktuatorschafts 9 in negative x-Richtung ist der Aktuatorschaft 9 an der Rückseite 17 des Zwischenrahmens durch die Mutter 11 gesichert. Dadurch führt eine Drehung des Aktuatorschafts dazu, dass sich der nicht um die Klemmrichtung x drehbare Aufspreizschlitten 7 durch die Gewindekopplung in x-Richtung innerhalb des Innenraums 13 verschiebt.

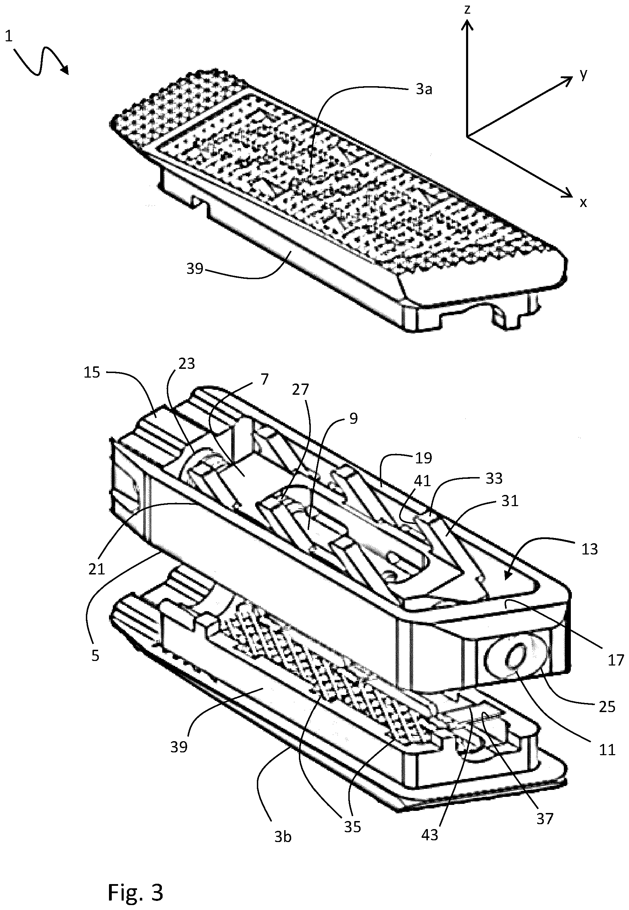

In der Explosionsansicht in Fig. 3 ist auch das untere Knochenanlageelement 3b nach unten hin, also in negative Z Richtung, abgehoben dargestellt. Dadurch wird die Wirkungsweise des Aufspreizmechanismus des Zwischenwirbelimplantats 1 deutlich. Der Aufspreizschlitten 7 weist insgesamt zwölf erste Gleitflächen 31 auf, von denen in Fig. 2 bis 4 nur die oberen sechs Gleitflächen 31 zu sehen sind. Jede der Gleitflächen 31 wird jeweils von einem in positive x-Richtung schräg nach unten verlaufenden Steg 33 gebildet. Die Hälfte der Stege 33 befinden sich an der ersten lateralen Seite 19 des Zwischenrahmens 5 und die andere Hälfte der Stege 33 befindet sich an der gegenüberliegenden zweiten lateralen Seite 21 des Zwischenrahmens 5. Die sichtbaren oberen sechs Stege 33 verlaufen alle parallel zueinander, sodass auch die sechs oberen ersten Gleitflächen 31 alle parallel zueinander ausgerichtet sind. In Fig. 3 sind in dem unteren Knochenanlageelement 3b sechs Nuten 35 erkennbar, die in ihrer Schrägneigung mit den unteren sechs Stegen 33 des Aktuatorschlittens 7 korrespondieren, sodass diese darin gleitend eingreifen. Das obere Knochenanlageelement 3a weist analog dazu entsprechende Nuten 35 auf, die in ihrer Schräglage mit den oberen sechs Stegen 33 des Aufspreizschlittens 7 korrespondieren, sodass diese darin gleitend gelagert sind. Die Nuten 35 bilden daher zu den ersten Gleitflächen 35 der Knochenanlageelemente 3a,b zweite Gleitflächen 37 aus, die beim Aufspreizen des Zwischenwirbelimplantats 1 auf den ersten Gleitflächen 33 gleiten. Die Nuten 35 sind hier innenseitig in lateralen Randleisten 39 der Knochenanlageelemente 3a,b ausgebildet. Die lateralen Randleisten 39 der Knochenanalageelemente 3a,b greifen zudem als Führungselement außenseitig passgenau in den Innenraum 13 des Zwischenrahmens 5 ein, sodass sich die Knochenanlageelemente 3a,b ausschließlich in Spreizrichtung Z relativ zum Zwischenrahmen 5 bewegen können. Durch die Schrägstellung der Gleitflächen 31, 37 wird eine Bewegung des Aufspreizschlittens 7 in positive x-Richtung in ein Auseinanderspreizen der Knochenanlageelemente 3a,b umgesetzt. Dies bedeutet, dass der Aufspreizschlitten 7 das obere Knochenanlageelement 3a nach oben drückt und das untere Knochenanlageelement 3b nach unten drückt. Die Bewegung des Aufspreizschlittens 7 in positive x-Richtung kann beispielsweise durch eine Rechtsdrehung des Aktuatorschafts 9 bewegt werden, wenn der Aktuatorschaft 9 mit dem Aufspreizschlitten 7 über ein Rechtsgewinde gekoppelt ist. Die als separaten Bauteile ausgebildeten Knochenanlageelemente 3a,b sind zudem durch die Stege 33 des Aufspreizschlittens 7 unverlierbar gekoppelt, solange der Aktuatorschaft 9 den Aufspreizschlitten 7 im Zwischenrahmen 5 sichert. Dies gelingt dadurch, dass die oberen Stege 33 unterseitig dritte Gleitflächen 41 aufweisen und die unteren Stege 33 oberseitig dritte Gleitflächen 41 aufweisen. Die Nuten 35 der Knochenanlageelemente 3a,b weisen entsprechende vierte Gleitflächen 43 auf, die mit den dritten Gleitflächen 41 korrespondieren und ein Zusammenziehen der Knochenanlageelemente 3a,b bewirken, wenn der Aufspreizschlitten 7 in negative x-Richtung bewegt wird. Dies kann durch eine Linksdrehung des Aktuatorschafts 9 bewirkt werden, wenn der Aktuatorschaft 9 und der Aufspreizschlitten 7 über ein Rechtsgewinde miteinander gekoppelt sind.

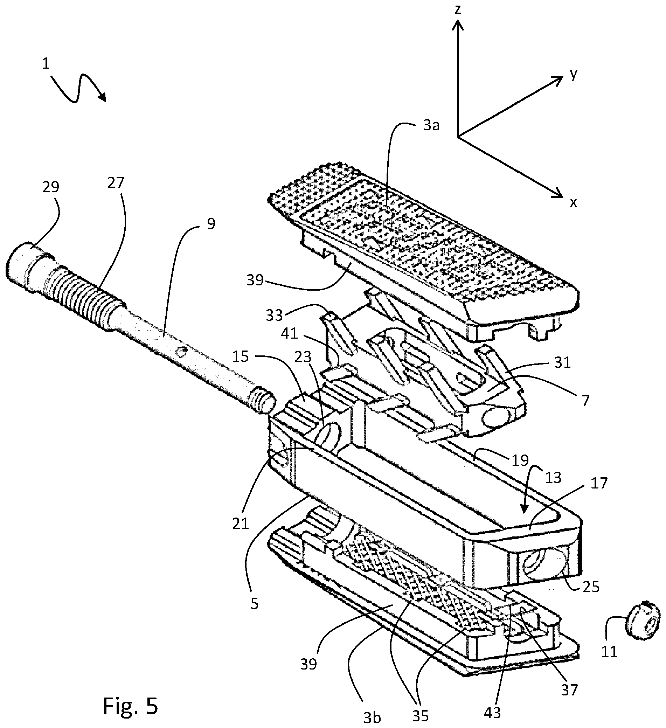

In Fig. 5 wird der im Wesentlichen sowohl bezüglich der xy-Ebene als auch bezüglich der xz-Ebene spiegelsymmetrische Aufbau des Aufspreizschlittens 7 deutlich. Lediglich an der Rückseite wird diese Symmetrie leicht durchbrochen, da die erste laterale Seite 19 des Zwischenrahmens 5 länger ist als die zweite laterale Seite 21. Die Rückseite 17 des Zwischenrahmens 5 verläuft also schräg zur Breitenrichtung y. Dies gilt ebenso für das rückseitige Ende der Knochenanlageelemente 3a,b. Dadurch kann die mit dem Wirbelknochen in Kontakt kommende Fläche der Knochenanlageseite der Knochenanlageelemente 3a,b möglichst groß ausgestaltet werden und zugleich das Zwischenwirbelimplantat 1 medio-lateral in einen Zwischenwirbelraum platziert werden.

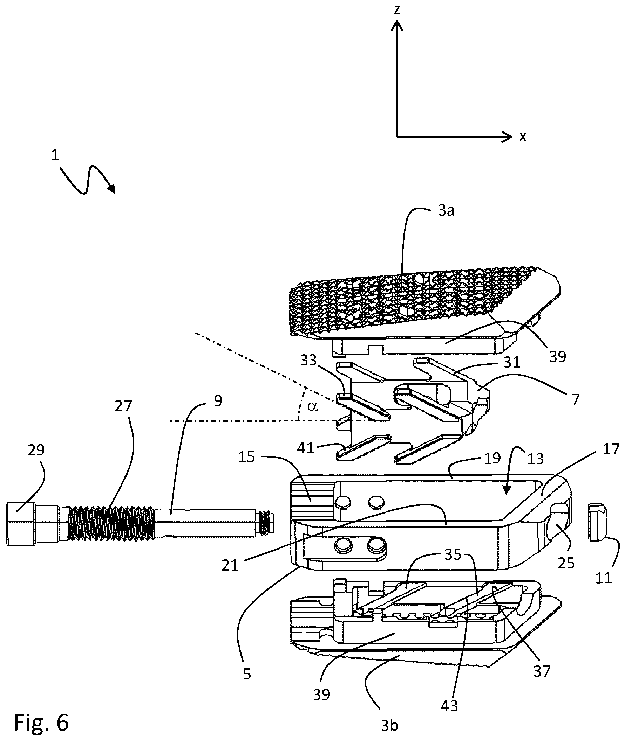

In Fig. 6 in eine etwas kürzere Ausführungsform eines Zwischenwirbelimplantats 1 gezeigt, dessen Aufspreizschlitten nur jeweils vier Stege 33 oben und unten aufweist, also acht erste Gleitflächen 31 und acht dritte Gleitflächen 41. Entsprechend weisen die Knochenanlageelemente 3a,b jeweils vier Stege 33 auf, welche die korrespondieren zweiten Gleitflächen 37 und vierten Gleitflächen 43 ausbilden. In Fig. 6 ist auch ein Winkel α gezeigt, in dem die Gleitflächen 31, 37, 41, 43 schräg zur Klemmrichtung x verlaufen und welcher je nach Modell des Zwischenwirbelimplantats 1 vorzugsweise im Bereich von 10° bis 45° liegt.

### Bezugszeichenliste

- 1: Zwischenwirbelimplantat
- 3a,b: Knochenanlageelemente
- 5: Zwischenrahmen
- 7: Aufspreizschlitten
- 9: Aktuatorschaft
- 11: Sicherungsmittel
- 13: Innenraum des Zwischenrahmens
- 15: Frontseite des Zwischenrahmens
- 17: Rückseite des Zwischenrahmens
- 19: erste laterale Seite des Zwischenrahmens
- 21: zweite laterale Seite des Zwischenrahmens
- 23: erste Aktuatorschaftaufnahmeöffnung
- 25: zweite Aktuatorschaftaufnahmeöffnung
- 27: Gewinde
- 29: Bolzenkopf
- 31: erste Gleitflächen
- 33: Stege
- 35: Nuten
- 37: zweite Gleitflächen
- 39: laterale Randleisten der Knochenanlageelemente
- 41: dritte Gleitflächen
- 43: vierte Gleitflächen
- α: Winkel
- x: Klemmrichtung
- z: Spreizrichtung
- y: Breitenrichtung

## Patentansprüche

1. Aufspreizbares Zwischenwirbelimplantat (1) mit:
- zwei relativ zueinander in einer Spreizrichtung (z) beweglichen Knochenanlageelementen (3a,b),
- einem zwischen den Knochenanlageelementen (3a,b) angeordneten Zwischenrahmen,
- einem innerhalb des Zwischenrahmens (5) in einer quer zur Spreizrichtung (z) verlaufenden Klemmrichtung (x) beweglichen Aufspreizschlitten (7), und
- einem in Klemmrichtung (x) durchgehend durch den Aufspreizschlitten (7) verlaufenden Aktuatorschaft (9),
wobei der Aufspreizschlitten (7) erste Gleitflächen (31) aufweist, welche schräg zur Klemmrichtung (x) verlaufen,
wobei jedes der Knochenanlageelemente (3a,b) mindestens eine zu einer der ersten Gleitflächen (31) korrespondierende zweite Gleitfläche (37) aufweist, welche parallel zu der korrespondierenden ersten Gleitfläche (31) verläuft und bei Klemmrichtungsbewegung des Aufspreizschlittens (7) auf dieser gleitet, wobei der Aktuatorschaft (9) an einer Frontseite des Zwischenrahmens (15) und an einer Rückseite des Zwischenrahmens (17) um die Klemmrichtung (x) drehbar gelagert und mit dem Aufspreizschlitten (7) derart formschlüssig gekoppelt ist, dass sich der Aufspreizschlitten (7) bei Drehung des Aktuatorschafts (9) zwischen der Frontseite des Zwischenrahmens (15) und der Rückseite des Zwischenrahmens (17) in Klemmrichtung (x) bewegt.

2. Aufspreizbares Zwischenwirbelimplantat (1) gemäß Anspruch 1, wobei die Knochenanlageelemente (3a,b) ausschließlich linear und rotationslos in der Spreizrichtung (z) beweglich sind.

3. Aufspreizbares Zwischenwirbelimplantat (1) gemäß einem der vorhergehenden Ansprüche, wobei der Aufspreizschlitten (7) von den ersten Gleitflächen (31) weg gerichtete dritte Gleitflächen (41) aufweist, wobei jedes der Knochenanlageelemente (3a,b) mindestens eine zu einer der dritten Gleitflächen (41) korrespondierende vierte Gleitfläche (43) aufweist, welche parallel zu der korrespondierenden dritten Gleitfläche (41) verläuft und bei Bewegung des Aufspreizschlittens (7) entgegen der Klemmrichtungsbewegung auf dieser gleitet.

4. Aufspreizbares Zwischenwirbelimplantat (1) gemäß einem der vorhergehenden Ansprüche, wobei die Frontseite des Zwischenrahmens (15) eine erste Aktuatorschaftaufnahmeöffnung (23) aufweist, und wobei die Rückseite des Zwischenrahmens (17) eine zweite Aktuatorschaftaufnahmeöffnung (25) aufweist.

5. Aufspreizbares Zwischenwirbelimplantat (1) gemäß einem der vorhergehenden Ansprüche, wobei der Aktuatorschaft (9) von der Frontseite des Zwischenrahmens (15) aus in den Zwischenrahmen (5) einsteckbar ist und ein durch die Rückseite des Zwischenrahmens (17) ragendes Ende des Aktuatorschafts (9) mit einem Sicherungsmittel (11) an der Rückseite des Zwischenrahmens (17) gesichert ist.

6. Aufspreizbares Zwischenwirbelimplantat (1) gemäß einem der vorhergehenden Ansprüche, wobei die Rückseite des Zwischenrahmens (17) beidseitig lateral und/oder zu den Knochenanlageelementen (3a,b) hin schräg angefasst ist.

7. Aufspreizbares Zwischenwirbelimplantat (1) gemäß einem der vorhergehenden Ansprüche, wobei der Zwischenrahmen (5) eine erste laterale Seite (19) aufweist, die zwischen der Frontseite und der Rückseite verläuft, und wobei der Zwischenrahmen (5) eine der ersten lateralen Seite (19) gegenüberliegende zweite laterale Seite (21) aufweist, die zwischen der Frontseite und der Rückseite verläuft, wobei die erste laterale Seite (19) in Klemmrichtung (x) kürzer ist als die zweite laterale Seite (21).

8. Aufspreizbares Zwischenwirbelimplantat (1) gemäß einem der vorhergehenden Ansprüche, wobei sich die Knochenanlageelemente (3a,b) jeweils zur Rückseite des Zwischenrahmens (17) hin keilförmig verdicken.

9. Aufspreizbares Zwischenwirbelimplantat (1) gemäß einem der vorhergehenden Ansprüche, wobei die Knochenanlageelemente (3a,b) jeweils durch ein generatives Schichtaufbauverfahren, vorzugsweise Selektives Lasersintern, SLS, aus Titan oder einem titanhaltigen Material hergestellt sind.

10. Aufspreizbares Zwischenwirbelimplantat (1) gemäß einem der vorhergehenden Ansprüche, wobei die Knochenanlageelemente (3a,b) jeweils eine Knochenanlageseite aufweisen, wobei die Knochenanlageseite ein Profil und/oder Löcher aufweist.

11. Aufspreizbares Zwischenwirbelimplantat (1) gemäß einem der vorhergehenden Ansprüche, wobei die ersten Gleitflächen (31) spiegelsymmetrisch zu einer quer zur Spreizrichtung (z) verlaufenden Ebene angeordnet sind.

12. Aufspreizbares Zwischenwirbelimplantat (1) gemäß einem der vorhergehenden Ansprüche, wobei eine Hälfte der ersten Gleitflächen (31) an einer Oberseite des Aufspreizschlittens (7) angeordnet ist, und wobei eine andere Hälfte der ersten Gleitflächen (31) an einer Unterseite des Aufspreizschlittens (7) angeordnet ist.

13. Aufspreizbares Zwischenwirbelimplantat (1) gemäß einem der vorhergehenden Ansprüche, wobei eine Hälfte der ersten Gleitflächen (31) an einer ersten lateralen Seite des Aufspreizschlittens (7) angeordnet ist, und wobei eine andere Hälfte der ersten Gleitflächen (31) an einer der ersten lateralen Seite gegenüberliegenden zweiten lateralen Seite des Aufspreizschlittens (7) angeordnet ist.

14. Aufspreizbares Zwischenwirbelimplantat (1) gemäß einem der vorhergehenden Ansprüche, wobei die zweiten Gleitflächen (37) durch innenseitig schräg in lateralen Randleisten der Knochenanlageelemente (39) verlaufende Nuten (35) ausgebildet sind.

15. Aufspreizbares Zwischenwirbelimplantat (1) gemäß Anspruch 14, wobei die lateralen Randleisten der Knochenanlageelemente (39) als Führungselement außenseitig passgenau in den Zwischenrahmen (5) eingreifen.

## Claims

1. An expandable intervertebral implant (1) having:
- two bone contact elements (3a, b) movable relative to one another in an expansion direction (z),
- an intermediate frame arranged between the bone contact elements (3a, b),
- an expansion slide (7) movable within the intermediate frame (5) in a clamping direction (x) running transverse to the expansion direction (z), and
- an actuator shaft (9) running continuously through the expansion slide (7) in the clamping direction (x),
wherein the expansion slide (7) has first slide faces (31), which run at an incline to the clamping direction (x),
wherein each of the bone contact elements (3a, b) has at least one second slide face (37), which corresponds to one of the first slide faces (31) and which runs parallel to the corresponding first slide face (31) and slides thereon when the expansion slide (7) moves in the clamping direction, wherein the actuator shaft (9) is mounted rotatably about the clamping direction (x) on a front side of the intermediate frame (15) and on a rear side of the intermediate frame (17) and is coupled form-fittingly to the expansion slide (7) in such a way that the expansion slide (7), upon rotation of the actuator shaft (9), moves in the clamping direction (x) between the front side of the intermediate frame (15) and the rear side of the intermediate frame (17).

2. The expandable intervertebral implant (1) according to claim 1, wherein the bone contact elements (3a, b) are movable exclusively linearly and without rotation in the expansion direction (z).

3. The expandable intervertebral implant (1) according to any one of the preceding claims, wherein the expansion slide (7) has third slide faces (41) directed away from the first slide faces (31), wherein each of the bone contact elements (3a, b) has at least one fourth slide face (43) corresponding to one of the third slide faces (41), the at least one fourth slide running parallel to the corresponding third slide face (41) and sliding on the expansion slide (7) upon movement of the latter against the clamping direction movement.

4. The expandable intervertebral implant (1) according to any one of the preceding claims, wherein the front side of the intermediate frame (15) has a first actuator shaft receiving opening (23), and wherein the rear side of the intermediate frame (17) has a second actuator shaft receiving opening (25).

5. The expandable intervertebral implant (1) according to any one of the preceding claims, wherein the actuator shaft (9) can be inserted from the front side of the intermediate frame (15) into the intermediate frame (5) and an end of the actuator shaft (9) protruding through the rear side of the intermediate frame (17) is secured with a securing means (11) on the rear side of the intermediate frame (17).

6. The expandable intervertebral implant (1) according to any one of the preceding claims, wherein the rear side of the intermediate frame (17) is chamfered at an incline on both sides laterally and/or towards the bone contact elements (3a, b).

7. The expandable intervertebral implant (1) according to any one of the preceding claims, wherein the intermediate frame (5) has a first lateral side (19), which runs between the front side and the rear side, and wherein the intermediate frame (5) has a second lateral side (21), which is opposite the first lateral side (19) and which runs between the front side and the rear side, wherein the first lateral side (19) is shorter than the second lateral side (21) in the clamping direction (x).

8. The expandable intervertebral implant (1) according to any one of the preceding claims, wherein the bone contact elements (3a, b) each thicken in a wedge shape towards the rear side of the intermediate frame (17).

9. The expandable intervertebral implant (1) according to any one of the preceding claims, wherein the bone contact elements (3a, b) are each produced by a generative layer build-up process, preferably selective laser sintering, SLS, from titanium or a titanium-containing material.

10. The expandable intervertebral implant (1) according to any one of the preceding claims, wherein the bone contact elements (3a, b) each have a bone contact side, wherein the bone contact side has a profile and/or holes.

11. The expandable intervertebral implant (1) according to any one of the preceding claims, wherein the first slide faces (31) are arranged mirror-symmetrical to a plane running transverse to the expansion direction (z).

12. The expandable intervertebral implant (1) according to any one of the preceding claims, wherein one half of the first slide faces (31) is arranged on an upper side of the expansion slide (7), and wherein another half of the first slide faces (31) is arranged on an underside of the expansion slide (7).

13. The expandable intervertebral implant (1) according to any one of the preceding claims, wherein one half of the first slide faces (31) is arranged on a first lateral side of the expansion slide (7), and wherein another half of the first slide faces (31) is arranged on a second lateral side of the expansion slide (7) opposite the first lateral side.

14. The expandable intervertebral implant (1) according to any one of the preceding claims, wherein the second slide faces (37) are formed by grooves (35) running on the inner side at an incline in lateral edge strips of the bone contact elements (39).

15. The expandable intervertebral implant (1) according to claim 14, wherein the lateral edge strips of the bone contact elements (39) engage as guide element on the outer side with an accurate fit in the intermediate frame (5).

## Revendications

1. Implant inter-vertébral écartable (1) comprenant :
- deux éléments d'appui osseux (3a, b) mobiles l'un par rapport à l'autre dans un sens d'écartement (z),
- un cadre intermédiaire disposé entre les éléments d'appui osseux (3a, b),
- un chariot d'écartement (7) mobile à l'intérieur du cadre intermédiaire (5) dans un sens de serrage (x) transversal au sens d'écartement (z), et
- un arbre actionneur (9) traversant le chariot d'écartement (7) dans le sens de serrage (x),
dans lequel le chariot d'écartement (7) présente des premières surfaces coulissantes (31) qui sont de biais par rapport au sens de serrage (x),
dans lequel chacun des éléments d'appui osseux (3a, b) présente au moins une deuxième surface coulissante (37) correspondant à une des premières surfaces coulissantes (31), qui passe parallèlement à la première surface coulissante (31) correspondante et coulisse sur celle-ci lors du mouvement dans le sens de serrage du chariot d'écartement (7), dans lequel l'arbre actionneur (9) est disposé rotatif autour du sens de serrage (x) sur une face avant du cadre intermédiaire (15) et sur une face arrière du cadre intermédiaire (17) et est couplé par adhérence de formes au chariot d'écartement (7) de telle façon que le chariot d'écartement (7), lors de la rotation de l'arbre actionneur (9) entre la face avant du cadre intermédiaire (15) et la face arrière du cadre intermédiaire (17), se déplace dans le sens de serrage (x).

2. Implant inter-vertébral écartable (1) selon la revendication 1, dans lequel les éléments d'appui osseux (3a, b) sont mobiles exclusivement de manière linéaire et sans rotation dans le sens d'écartement (z).

3. Implant inter-vertébral écartable (1) selon l'une des revendications précédentes, dans lequel le chariot d'écartement (7) présente des troisièmes surfaces coulissantes (41) orientées en s'éloignant des premières surfaces coulissantes (31), dans lequel chacun des éléments d'appui osseux (3a, b) présente au moins une quatrième surface coulissante (43) correspondant à un des troisièmes surfaces coulissantes (41), qui passe parallèlement à la troisième surface coulissante (41) correspondante et coulisse sur celle-ci lors du mouvement du chariot d'écartement (7) contre le mouvement dans le sens de serrage.

4. Implant inter-vertébral écartable (1) selon l'une des revendications précédentes, dans lequel la face avant du cadre intermédiaire (15) présente une première ouverture de réception d'arbre actionneur (23) et dans lequel la face arrière du cadre intermédiaire (17) présente une seconde ouverture de réception d'arbre actionneur (25).

5. Implant inter-vertébral écartable (1) selon l'une des revendications précédentes, dans lequel l'arbre actionneur (9) peut être inséré de la face avant du cadre intermédiaire (15) dans le cadre intermédiaire (5) et une extrémité de l'arbre actionneur (9) faisant saillie à travers la face arrière du cadre intermédiaire (17) est fixée par un moyen de fixation (11) sur la face arrière du cadre intermédiaire (17).

6. Implant inter-vertébral écartable (1) selon l'une des revendications précédentes, dans lequel la face arrière du cadre intermédiaire (17) est prise des deux côtés latéralement et/ou de biais en direction des éléments d'appui osseux (3a, b).

7. Implant inter-vertébral écartable (1) selon l'une des revendications précédentes, dans lequel le cadre intermédiaire (5) présente un premier côté latéral (19) qui passe entre la face avant et la face arrière, et dans lequel le cadre intermédiaire (5) présente un second côté latéral (21) faisant face au premier côté latéral (19), qui passe entre la face avant et la face arrière, dans lequel le premier côté latéral (19) est plus court dans le sens de serrage (x) que le second côté latéral (21).

8. Implant inter-vertébral écartable (1) selon l'une des revendications précédentes, dans lequel les éléments d'appui osseux (3a, b) s'épaississent respectivement de manière cunéiforme en direction de la face arrière du cadre intermédiaire (17).

9. Implant inter-vertébral écartable (1) selon l'une des revendications précédentes, dans lequel les éléments d'appui osseux (3a, b) sont fabriqués respectivement par un procédé de montage de couches génératif, de préférence par frittage sélectif par laser, SLS, en titane ou dans un matériau contenant du titane.

10. Implant inter-vertébral écartable (1) selon l'une des revendications précédentes, dans lequel les éléments d'appui osseux (3a, b) présentent respectivement un côté d'appui osseux, dans lequel le côté d'appui osseux présente un profilé et/ou des trous.

11. Implant inter-vertébral écartable (1) selon l'une des revendications précédentes, dans lequel les premières surfaces coulissantes (31) sont disposées en symétrie de miroir par rapport à un plan transversal au sens d'écartement (z).

12. Implant inter-vertébral écartable (1) selon l'une des revendications précédentes, dans lequel une moitié des premières surfaces coulissantes (31) est disposée sur une face supérieure du chariot d'écartement (7), et dans lequel une autre moitié des premières surfaces coulissantes (31) est disposée sur une face inférieure du chariot d'écartement (7).

13. Implant inter-vertébral écartable (1) selon l'une des revendications précédentes, dans lequel une moitié des premières surfaces coulissantes (31) est disposée sur une première face latérale du chariot d'écartement (7), et dans lequel une autre moitié des premières surfaces coulissantes (31) est disposée sur une seconde face latérale du chariot d'écartement (7) faisant face à la première face latérale.

14. Implant inter-vertébral écartable (1) selon l'une des revendications précédentes, dans lequel les deuxièmes surfaces coulissantes (37) sont formées par des encoches (35) passant intérieurement en biais dans des baguettes de bordure latérales des éléments d'appui osseux (39).

15. Implant inter-vertébral écartable (1) selon la revendication 14, dans lequel les baguettes de bordure latérales des éléments d'appui osseux (39) se mettent en prise avec une précision ajustée sur l'extérieur dans le cadre intermédiaire (5) en tant qu'élément de guidage.
